(19) Europäisches Patentamt
European Patent Office
Office européen des brevets

(11) EP 2 673 261 B1

(12) FASCICULE DE BREVET EUROPEEN

(45) Date de publication et mention de la délivrance du brevet:
**16.12.2015 Bulletin 2015/51**

(21) Numéro de dépôt: **12702280.4**

(22) Date de dépôt: **07.02.2012**

(51) Int Cl.:
***C07D 213/30*** (2006.01) ***C07D 277/24*** (2006.01)
***C07D 333/16*** (2006.01) ***A61Q 5/08*** (2006.01)
***A61Q 19/02*** (2006.01) ***A61Q 19/08*** (2006.01)
***A61Q 17/00*** (2006.01)

(86) Numéro de dépôt international:
**PCT/EP2012/051998**

(87) Numéro de publication internationale:
**WO 2012/107421 (16.08.2012 Gazette 2012/33)**

# (54) DERIVES HETEROCYCLIQUES DU RESORCINOL, LEUR PREPARATION ET LEURS UTILISATIONS COSMETIQUES

HETEROCYCLISCHE RESORCINOLDERIVATE, IHRE HERSTELLUNG UND IHRE VERWENDUNG IN DER KOSMETIK

HETEROCYCLIC RESORCINOL DERIVATIVES, PREPARATION OF SAME AND COSMETIC USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.02.2011 FR 1150957**

(43) Date de publication de la demande:
**18.12.2013 Bulletin 2013/51**

(73) Titulaire: **Pierre Fabre Dermo-Cosmétique**
**92100 Boulogne-Billancourt (FR)**

(72) Inventeurs:
- **POIGNY, Stéphane**
**F-31600 Saubens (FR)**
- **BELAUBRE, Françoise**
**F-31270 Villeneuve Tolosane (FR)**

(74) Mandataire: **Regimbeau**
**20, rue de Chazelles**
**75847 Paris Cedex 17 (FR)**

(56) Documents cités:
**US-A1- 2007 098 655 US-A1- 2010 016 347**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 673 261 B1

## Description

La présente invention concerne de nouveaux analogues du résorcinol, leurs utilisations, et leurs procédés de préparation.

La présente invention concerne des nouveaux analogues du résorcinol correspondant à la formule générale (I) :

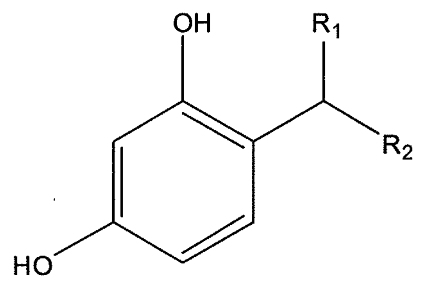

(I)

dans laquelle :

- R1 est un groupement méthyle,
- et R2 représente un monocycle hétéro aromatique, azoté, soufré ou oxygéné, de préférence à 5 ou 6 chaînons

Le Résorcinol (ou benzène 1,3-diol) de formule suivante :

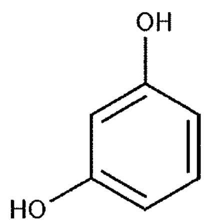

est connu pour ces utilisations diverses en tant qu'antiseptique mais aussi et surtout en tant que colorant ; tels que le *bleu de résorcine* ou le *vert de résorcine.*

Ces derniers sont utilisés en tant qu'indicateurs colorés dans certains titrages acido-basiques.

Des analogues du résorcinol ont été décrits dans la demande de brevet d'Unigen Pharmaceuticals Inc. WO 2010/011630 et ont une formule générale apparentée à celle de la présente invention, à savoir :

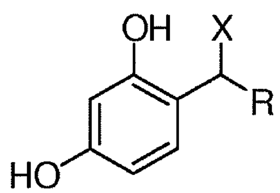

dans laquelle X peut être soit un atome d'hydrogène, soit un radical -OH, et R est un noyau aromatique ou hétéroaromatique ; ce en quoi la présente invention diffère en proposant des dérivés de formules chimiques différentes.

L'état de la technique peut être illustré par les documents US 2010/016347 et US 2007/098655 qui décrivent des inhibiteurs de tyrosinase ayant des propriétés dépigmentantes qui répondent à des formules distinctes de celles des composés de la présente invention.

Le problème que se propose de résoudre la présente invention est l'inhibition de la synthèse de mélanine, par l'inhibition de l'activité tyrosinase (activité au coeur de la synthèse de mélanine), et par l'utilisation des ces nouveaux analogues du résorcinol, dans le but de parvenir à une dépigmentation de la peau efficace.

En outre l'utilisation de ces analogues du résorcinol se propose de lutter contre le vieillissement de la peau et ce, entre autre, par une activité anti-oxydante élevée de ces composés.

L'épithélium de la peau, formé de l'épiderme et du derme, donne sa couleur à la peau, principalement grâce à certaines cellules : les mélanocytes qui produisent de la mélanine qui est composée de plusieurs pigments.

La tyrosinase est une enzyme limitante dans la mélanogénèse, dont l'inhibition entraîne une dépigmentation. Elle appartient à la famille des oxydoréductases.

La tyrosinase possède notamment la fonction : monophénol mono oxygénase (MPMO) et polyphénol oxydase (PPO).

La tyrosinase est synthétisée au niveau des mélanocytes. Elle est activée lors de sa migration vers les kératinocytes via les mélanosomes. Elle transforme la tyrosine en DOPA puis dopaquinone, ce qui conduit à une polymérisation soit une production de pigments. (Voir schéma ci-dessous)

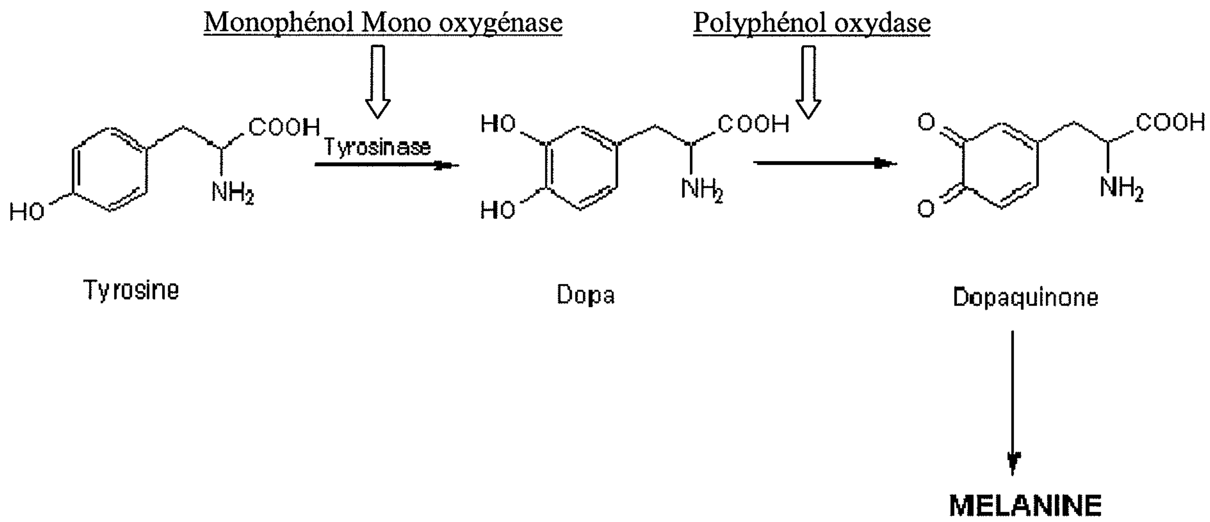


Du point de vue de l'art antérieur, la demande de brevet d'Unigen Pharmaceuticals Inc. WO 2010/011630, a démontré que les dérivés du résorcinol revendiqués dans cette demande étaient dotés d'une certaine activité d'inhibition de l'activité tyrosinase, d'inhibition de la production de mélanine et d'un pouvoir blanchissant sur la peau.

Ce en quoi, l'inventeur de la présente invention a apporté une réponse en termes d'efficacité supérieure à celle décrite pour les composés revendiqués de l'art antérieur. Ces composés se révélant par la même occasion de meilleurs agents dépigmentants.

La présente invention concerne donc des nouveaux analogues du résorcinol correspondant à la formule générale (I) :

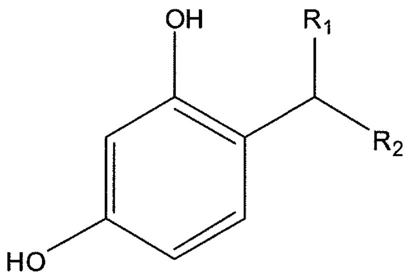

(I)

dans laquelle :

R1 = Alkyle en C1 à C4 et R2 = cycle hétéro aromatique azoté, soufré ou oxygéné en particulier un monocycle hétéro aromatique, de préférence un cycle hétéro aromatique à 5 ou 6 chaînons,
ledit cycle pouvant être optionnellement substitué par un radical alkyle linéaire ou ramifié en $C_1$ à $C_4$, de préférence un radical méthyle,
et ayant pour radicaux préférés, les radicaux suivants : R1 = Méthyle et R2 choisi parmi 3-pyridyl, 4-pyridyl, 2-furyl, 3-furyl, 2-thiophényl, 3-thiophényl, 2-thiazolyl.

Selon un mode de réalisation de l'invention les composés de formule générale (I) peuvent être choisis parmi les composés suivants:

- 4-(1-(pyridin-2-yl)éthyl)benzène-1,3-diol, = Composé 2
- 4-(1-(pyridin-3-yl)éthyl)benzène-1,3-diol, = Composé 3
- 4-(1-(pyridin-4-yl)éthyl)benzène-1,3-diol,
- 4-(1-(thiophène-2-yl)éthyl)benzène-1,3-diol, = Composé 1
- 4-(1-(thiophène-3-yl)éthyl)benzène-1,3-diol, = Composé 4
- 4-(1-(thiazol-2-yl)éthyl)benzène-1,3-diol, = Composé 5
- 4-(1-(1-méthyl-1H-benzo[d]imidazol-2-yl)éthyl)benzène-1,3-diol,
- 4-(1-(1-méthyl-1H-indol-2-yl)éthyl)benzène-1,3-diol,
- 4-(1-(benzo[b]thiophène-2-yl)éthyl)benzène-1,3-diol,
- 4-(1-(thiophène-2-yl)butyl)benzène-1,3-diol, = Composé 31
- 4-(3-méthyl-1-(thiophène-2-yl)butyl)benzène-1,3-diol, = Composé 28

L'invention concerne également l'utilisation cosmétique des composés de formule (I), et en particulier leur utilisation pour la dépigmentation de la peau, pour la mise en oeuvre d'une méthode de traitement cosmétique du vieillissement cutané.

La présente invention concerne l'utilisation cosmétique des composés de formule générale (I) en tant que principe actif antioxydant ou en tant que principe actif dépigmentant.

L'invention vise également les compositions pharmaceutiques ou cosmétiques comprenant au moins un des composés de formule (I) en association avec au moins un excipient pharmaceutiquement ou cosmétiquement acceptable.

Dans la présente invention, on entend désigner par «pharmaceutiquement ou cosmétiquement acceptable » ce qui est utile dans la préparation d'une composition pharmaceutique ou cosmétique, qui est généralement sûr, non toxique et ni biologiquement ni autrement non souhaitable et qui est acceptable pour une utilisation thérapeutique ou cosmétique, notamment par application topique.

L'objet de l'invention concerne une composition cosmétique caractérisée en ce que la quantité de composé de formule (I) varie entre 0,01 % et 10% et de préférence de 0,1 % à 5% en poids par rapport au poids total de la composition.

La présente invention concerne un procédé de blanchiment et/ou d'éclaircissement de la peau humaine et/ou des poils et/ou des cheveux comprenant l'application sur la peau et/ou les poils et/ou les cheveux d'une composition cosmétique contenant au moins un composé de formule (I).

La présente invention concerne une méthode de traitement et/ou de prévention cosmétique du vieillissement de la peau comprenant l'application sur la peau d'une composition cosmétique contenant au moins un composé de formule (I).

L'objet de l'invention s'étend également au procédé de synthèse des nouveaux composés de formule (I).

La présente invention sera mieux comprise en regard des exemples donnés ci-après à simple titre illustratif.

<u>Exemple 1 :</u>

<u>Synthèse du Composé 1 : 4-(1-(thiophène-2-yl)éthyl)benzène-1,3-diol</u>

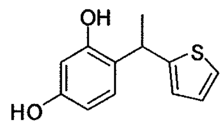

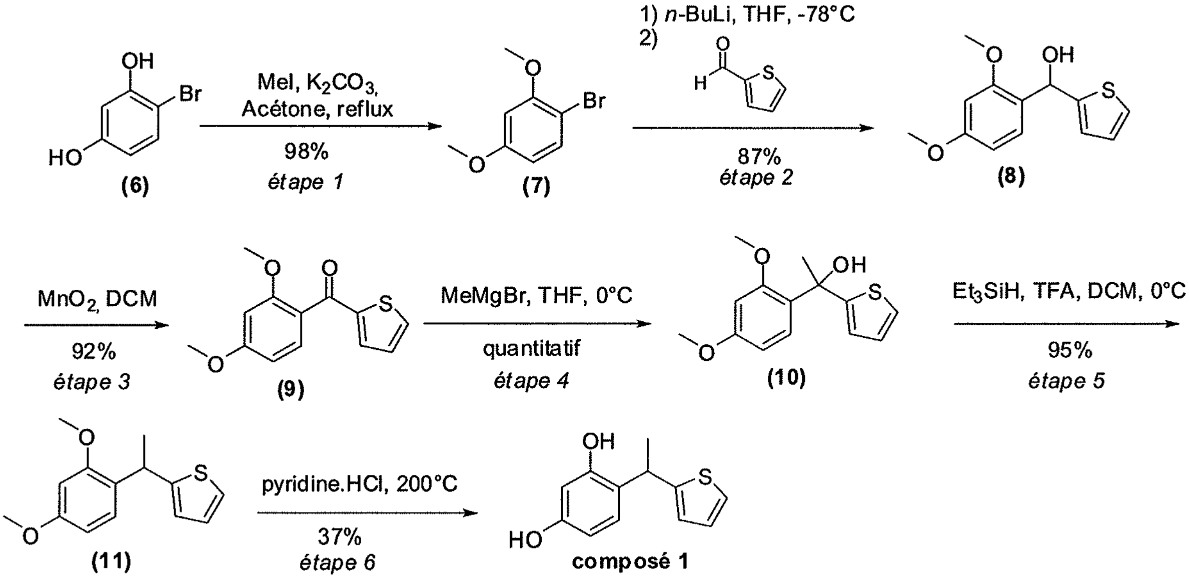

(6) (7) (8) (9) (10) (11) **composé 1**

<u>Synthèse du composé (7) :</u> 1-bromo-2,4-diméthoxybenzène

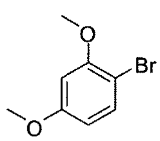

A une solution du composé (6) (10 g ; 52,9 mmol ; 1,0 équiv.) dans 200mL d'acétone sont additionnés successivement du carbonate de potassium (22 g ; 158,7 mmol ; 3,0 équiv.) et du iodométhane (9,9 mL ; 158,7 mmol ; 3,0 équiv.). Le mélange réactionnel est agité au reflux pendant 20h puis évaporé sous pression réduite. Le résidu obtenu est repris dans 200 mL d'eau et extrait à l'acétate d'éthyle (2 x 200 mL). Les phases organiques sont regroupées, séchées sur $MgSO_4$ puis évaporées pour conduire au composé (7) (11,2 g ; 98%) obtenu sous la forme d'une huile jaune. RMN $^1H$ (300 MHz, $CDCl_3$) : δ : 3,83 (s, 3H) ; 3,89 (s, 3H) ; 6,42 (dd, 1H) ; 6,50 (d, 1H) ; 7,42 (d, 1H).

<u>Synthèse du composé (8) :</u> (2,4-diméthoxyphényl)(thiophène-2-yl)méthanol

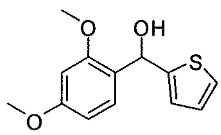

A une solution du composé (7) (3 g ; 13,8 mmol ; 1,0 équiv.) dans 90 mL de THF anhydre refroidie à -78°C est additionnée au goutte à goutte une solution de *n*-butyllithium 1,6 M dans l'hexane (9,5 mL ; 15,2 mmol ; 1,1 équiv.). La solution est agitée 1h à -78°C puis le 2-formylthiophène (4,7 mL ; 49,5 mmol ; 3,6 équiv.) est additionné au goutte au goutte. La solution est agitée 3h à 78°C, 15h à température ambiante puis 150 mL d'eau sont additionnés. Après extraction par de l'acétate d'éthyle (2 x 150 mL), les phases organiques sont regroupées, séchées sur $MgSO_4$ puis concentrées sous pression réduite. L'huile obtenue est purifiée par chromatographie sur gel de silice (éluant cyclohexane/acétate d'éthyle : 10/0 à 8/2), pour conduire à un mélange du composé (8) et du thiophène-2-méthanol (3,3 g ; 85%

massique de composé (8) ; 87%) obtenu sous la forme d'une huile jaune.
RMN $^1$H (300 MHz, $CD_3COCD_3$) : δ : 3,80 (s, 3H) ; 3,82 (s, 3H) ; 4,83 (d, 1H) ; 6,28 (dd, 1H) ; 6,54 (m, 2H) ; 6,83 (dd, 1H) ; 6,89 (dd, 1H) ; 6,97 (dd, 1H) ; 7,26 (dd, 1H) ; 7,43 (d, 1H).

Synthèse du composé (9) : (2,4-diméthoxyphényl)(thiophène-2-yl)méthanone

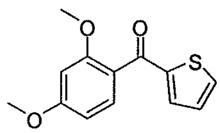

A une solution du composé (8) (3,3 g (85% massique) ; 11,24 mmol ; 1,0 équiv.) dans 100 mL de dichlorométhane est additionné du $MnO_2$ (6,0 g ; 69,0 mmol ; 6,1 équiv.). Le mélange réactionnel est agité 2 jours à température ambiante puis filtré sur célite. Le filtrat est concentré sous pression réduite pour conduire à un mélange du composé (8) et du composé (9). Ce mélange est remis en réaction dans les mêmes conditions pendant 2 jours. Après filtration sur célite et évaporation à sec, le résidu est purifié par chromatographie sur gel de silice (éluant cyclohexane/acétate d'éthyle : 10/0 à 9/1) pour conduire au composé (9) (2,55 g ; 92%) obtenu sous la forme d'une huile jaune.
RMN $^1$H (300 MHz, $CD_3COCD_3$) : δ : 3,80 (s, 3H) ; 3,90 (s, 3H) ; 6,65 (dd, 1H) ; 6,70 (d, 1H) ; 7,19 (dd, 1H) ; 7,37 (d, 1H) ; 7,49 (dd, 1H) ; 7,90 (dd, 1H).

Synthèse du composé (10) : 1-(2,4-diméthoxyphényl)-1-(thiophène-2-yl)éthanol

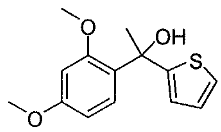

A une solution du composé (9) (2,6 g ; 10,3 mmol ; 1,0 équiv.) dans 100 mL de THF anhydre refroidie à 0°C est additionnée au goutte à goutte une solution du magnésien du bromométhane 3M dans l'éther diéthylique (10,3 mL ; 30,9 mmol ; 3,0 équiv.). Le mélange réactionnel est agité 2h à 0°C puis 5 mL d'une aqueuse de soude 2M et 100 mL d'eau sont additionnés successivement. Après extraction par de l'acétate d'éthyle (2 x 100 mL), les organiques sont regroupées, séchées sur $MgSO_4$ puis concentrées sous pression réduite pour conduire au composé (10) (2,7 g ; quantitatif) obtenu sous la forme d'une huile jaune.
RMN $^1$H (300 MHz, $CD_3COCD_3$) : δ : 1,78 (s, 3H) ; 3,59 (s, 3H) ; 3,67 (s, 3H) ; 4,79 (s, 1H) ; 6,39 (dd, 1H) ; 6,44 (d, 1H) ; 6,61 (d, 1H) ; 6,72 (dd, 1H) ; 7,09 (dd, 1H) ; 7,27 (d, 1H).

Synthèse du composé (11) : 2-(1-(2,4-diméthoxyphényl)éthyl)thiophènee

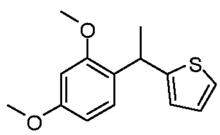

A une solution du composé (10) (2,7 g ; 10,3 mmol ; 1,0 équiv.) et de triéthylsilane (6,65 mL ; 41,2 mmol ; 4,0 équiv.) dans 60 mL de dichlorométhane anhydre refroidie à 0°C est additionné au goutte à goutte du TFA (0,84 mL ; 11,3 mmol; 1,1 équiv.). Le mélange réactionnel est agité 2h à 0°C puis 30 mL d'une aqueuse saturée en $NaHCO_3$ et 100 mL d'eau sont additionnés successivement. Les phases sont séparées puis la phase aqueuse est extraite par du dichlorométhane (100 mL). Les phases organiques sont regroupées, séchées sur $MgSO_4$ puis concentrées sous pression réduite pour conduire au composé (11) (2,4 g ; 95%) obtenu sous la forme d'une huile jaune.

RMN $^{1}H$ (300 MHz, $CD_3COCD_3$) : δ : 1,45 (d, 3H) ; 3,64 (s, 3H) ; 3,70 (s, 3H) ; 4,54 (q, 1H) ; 6,34 (dd, 1H) ; 6,41 (d, 1H) ; 6,71 (dd, 1H) ; 6,77 (dd, 1H) ; 6,92 (d, 1H) ; 7,07 (dd, 1H).

Synthèse du composé 1 : 4-(1-(thiophène-2-yl)éthyl)benzène-1,3-diol

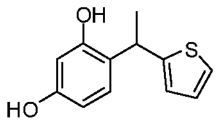

Un mélange du composé (11) (1,0 g ; 4,0 mmol ; 1,0 équiv.) et de chlorhydrate de pyridinium (30 g) est chauffé à 200°C pendant 1h30. Après refroidissement à température ambiante, de l'eau (150 mL), de l'acétate d'éthyle (75 mL) et une solution aqueuse d'HCl 2M (50 mL) sont additionnés. Les phases sont séparées puis la phase aqueuse est extraite par de l'acétate d'éthyle (2 x 100 mL). Les phases organiques sont regroupées, séchées sur $MgSO_4$ puis concentrées sous pression réduite. Le résidu est purifié par chromatographie sur gel de silice (éluant cyclohexane/acétate d'éthyle : 8/2 ; trois passages sur colonne sont nécessaires pour obtenir une pureté acceptable) pour conduire au composé 1 (0,33 g ; 37%) obtenu sous la forme d'une huile beige.
RMN $^{1}H$ (300 MHz, DMSO) : δ : 1,51 (d, 3H) ; 4,54 (q, 1H) ; 6,16 (m, 1H) ; 6,27 (d, 1H) ; 6,81 (m, 2H) ; 6,90 (dd, 1H) ; 7,27 (dd, 1H) ; 9,05 (s, 1H) ; 9,28 (s, 1H).
RMN $^{13}C$ (300 MHz, DMSO) : δ : 22,16 ; 32,06 ; 102,37; 106,29; 123,08; 126,40; 127,67; 129,76; 151,34; 154,84; 156,47; 158,45.
MS (EI) : 204,9; 219,94; 221,01

Exemple 2 :

Synthèse du composé 2 : 4-(1-(pyridin-2-yl)éthyl)benzène-1,3-diol

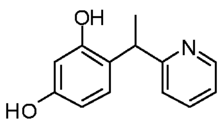

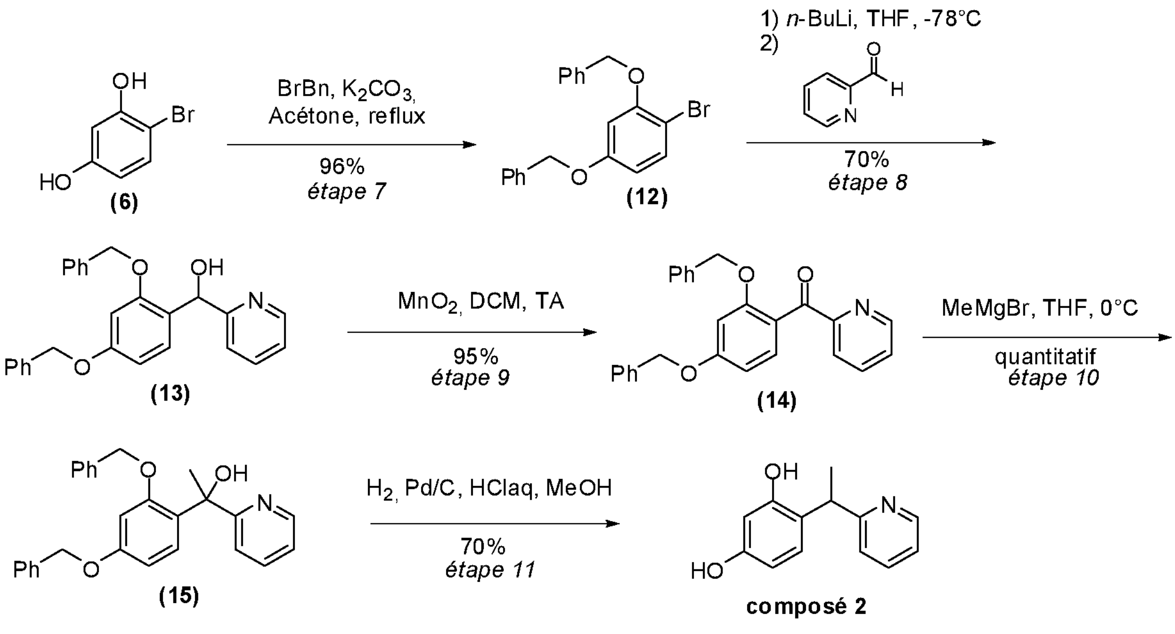

(6) (12) (13) (14) (15) composé 2

Synthèse du composé (12) : (4-bromo-1,3-phénylène)bis(oxy)bis(méthylène)dibenzène

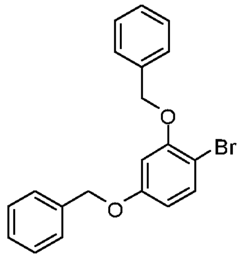

A une solution du composé (6) (5 g ; 26,5 mmol ; 1,0 équiv.) dans 100 mL d'acétone sont additionnés successivement du carbonate de potassium (11 g ; 79,5 mmol ; 3,0 équiv.) et du bromure de benzyle (9,5 mL ; 79,5 mmol ; 3,0 équiv.). Le mélange réactionnel est agité au reflux pendant 20h puis évaporé sous pression réduite. Le résidu obtenu est repris dans 100 mL d'eau et extrait par de l'acétate d'éthyle (2 x 100 mL). Les phases organiques sont regroupées, séchées sur $MgSO_4$ puis évaporées. L'huile obtenue est purifiée par chromatographie sur gel de silice (éluant : cyclohexane/acétate d'éthyle : 10/0 puis 9/1) pour conduire au composé (12) (9,4 g ; 96%) obtenu sous la forme d'un solide blanc.
RMN $^1H$ (300 MHz, $CDCl_3$): $\delta$ : 4,95 (s, 2H) ; 5,06 (s, 2H) ; 6,43 (dd, 1H) ; 6,55 (d, 1H) ; 7,21-7,40 (m, 11H).

Synthèse du composé (13) : (2,4-bis(benzyloxy)phényl)(pyridin-2-yl)méthanol

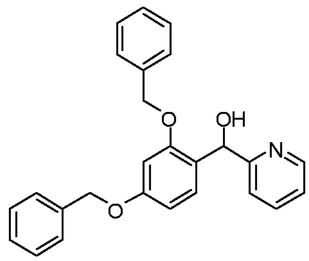

A une solution du composé (12) (5 g ; 13,5 mmol ; 1,0 équiv.) dans 130 mL de THF anhydre refroidie à -78°C est additionné au goutte à goutte une solution de *n*-butyllithium 1,6M dans l'hexane (9,3 mL ; 14,9 mmol ; 1,1 équiv.). La solution est agitée 1h à -78°C puis la 2-formylpyridine (4,5 mL ; 47,3 mmol ; 3,5 équiv.) est additionnée au goutte au goutte. La solution est agitée 3h à -78°C, 15h à température ambiante puis 800 mL d'eau sont additionnés. Le précipité obtenu est filtré sur Büchner, séché sous vide puis purifié par chromatographie sur gel de silice (éluant : cyclohexane/acétate d'éthyle : 7/3) pour conduire au composé (13) (3,65 g ; 70%) obtenu sous la forme d'un solide beige.
RMN $^1H$ (300 MHz, $CD_3COCD_3$) : $\delta$ : 5,13 (m, 3H) ; 5,16 (s, 2H) ; 6,16 (d, 1H) ; 6,61 (dd, 1H) ; 6,76 (d, 1H) ; 7,27-7,47 (m, 13H) ; 7,70 (ddd, 1H) ;.8,51 (d, 1H).

Synthèse du composé (14) : (2,4-bis(benzyloxy)phényl)(pyridin-2-yl)méthanone

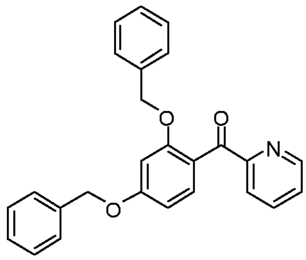

A une solution du composé (13) (3,6 g ; 9,4 mmol ; 1,0 équiv.) dans 70 mL de dichlorométhane est additionné du $MnO_2$ (4,9 g; 56,4 mmol ; 6,0 équiv.). Le mélange réactionnel est agité 4 jours à température ambiante puis filtré sur célite. Le filtrat est concentré sous pression réduite pour conduire au composé (14) (3,4 g ; 95%) obtenu sous la forme d'un solide blanc.
RMN $^1H$ (300 MHz, $CD_3COCD_3$) : $\delta$ : 5,00 (s, 2H) ; 5,24 (s, 2H) ; 6,79 (dd, 2H) ; 6,84 (s, 2H) ; 7,21 (m, 3H) ; 7,43 (m, 7H) ; 7,83 (dd, 1H) ;.7,92 (ddd, 1H) ; 8,56 (dd, 1H).

Synthèse du composé (15) : 1-(2,4-bis(benzyloxy)phényl)-1-(pyridin-2-yl)éthanol

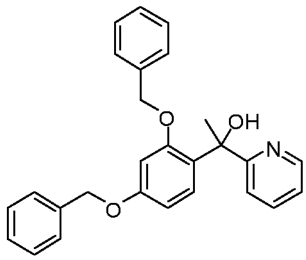

A une solution du composé (14) (3,35 g ; 8,8 mmol ; 1,0 équiv.) dans 70 mL de THF anhydre refroidie à 0°C est additionnée au goutte à goutte une solution du magnésien du bromométhane 3M dans l'éther diéthylique (8,8 mL ; 26,4 mmol ; 3,0 équiv.). Le mélange réactionnel est agité 2h à 0°C puis 5 mL d'une aqueuse de soude 2M et 100 mL d'eau sont additionnés successivement. Après extraction par de l'acétate d'éthyle (2 x 100 mL), les phases organiques sont regroupées, séchées sur $MgSO_4$ puis concentrées sous pression réduite pour conduire au composé (15) (3,5 g ; quantitatif) obtenu sous la forme d'un solide blanc.
RMN $^1H$ (300 MHz, $CD_3COCD_3$) : $\delta$ : 1,84 (s, 3H) ; 4,90 (dd ABsyst, 2H) ; 5,04 (s, 1H) ; 5,10 (s, 2H) ; 6,65 (m, 2H) ; 7,08 (m, 2H) ; 7,19 (dd, 1H) ; 7,30-7,43 (m, 9H) ; 7,47 (m, 2H) ;. 8,39 (dd, 1H).

Synthèse du composé 2 : 4-(1-(pyridin-2-yl)éthyl)benzène-1,3-diol

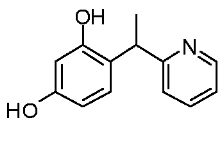

A une solution du composé (15) (3,5 g; 8,8 mmol, 1,0 équiv.) dans un mélange méthanol/ solution aqueuse d'HCl 2M (80 mL/20 mL) est additionné du Pd/C (10%, 50% wet) (1,0 g ; 28% massique). La suspension est agitée 4 jours sous atmosphère d'hydrogène puis filtrée sur Büchner. Le filtrat est évaporé sous pression réduite pour conduire à un mélange de composé 2 et d'alcène (28) sous forme de chlorhydrates. Le résidu est mis en solution dans un mélange méthanol/solution aqueuse d'HCl 2M (80 mL/20 mL) puis du Pd/C (10%, 50% wet) (1,0 g ; 28% massique) est additionné. La suspension est agitée 1 jour sous atmosphère d'hydrogène puis filtrée sur Büchner. Le filtrat est évaporé sous pression réduite. Le résidu obtenu est repris par une solution aqueuse saturée en $NaHCO_3$ (100 mL) puis extrait par de l'acétate d'éthyle (2 x 150 mL). Les phases organiques sont regroupées, séchées sur $MgSO_4$ puis concentrées sous pression réduite. Après purification par chromatographie sur gel de silice, le composé 2 est obtenu sous la forme d'une

mousse. Cette mousse est reprise par 100 mL d'éthanol puis l'éthanol est évaporé (opération répétée 3 fois) pour conduire au composé 2 (1,3 g ; 70%) obtenu sous la forme d'un solide beige.
RMN $^1H$ (300 MHz, DMSO) : δ : 1,50 (d, 3H) ; 4,39 (q, 1H) ; 6,17 (dd, 1H) ; 6,26 (d, 1H) ; 6,88 (d, 1H) ; 7,16 (m, 2H) ; 7,65 (ddd, 1H) ; 8,48 (d, 1H) ; 9,04 (s, 1H) ; 9,66 (s, 1H).
RMN $^{13}C$ (300 MHz, DMSO) : δ :19,83; 39,4; 102,72, 106,13; 121,11; 121,69; 121,92; 128,39; 136,61, 148,29; 155,40; 156,5; 165,11.
MS(EI): 198,0; 215,0.

<u>Exemple 3 :</u>

<u>Synthèse du composé 3 : 4-(1-(pyridin-3-yl)éthyl)benzène-1,3-diol</u>

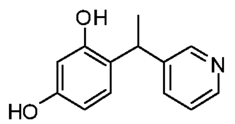

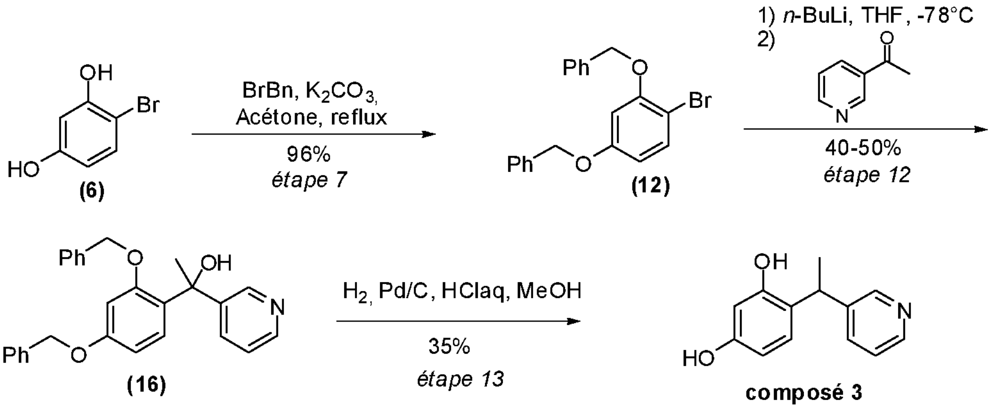

(6) (12) (16) composé 3

<u>Synthèse du composé (16) :</u> 1-(2,4-bis(benzyloxy)phényl)-1-(pyridin-3-yl)éthanol

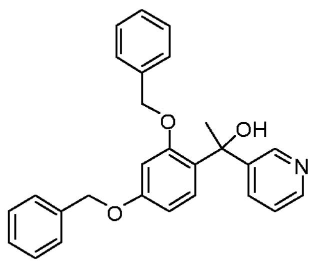

A une solution du composé (12) (8 g ; 21,7 mmol ; 1,0 équiv.) dans 300 mL de THF anhydre refroidie à -78°C est additionnée au goutte à goutte une solution de *n*-butyllithium 1,6M dans l'hexane (14,9 mL ; 23,8 mmol ; 1,1 équiv.). La solution est agitée 1h à -78°C puis la 3-acétylpyridine (2,6 mL ; 23,8 mmol ; 1,1 équiv.) est additionnée au goutte au goutte. La solution est agitée 3h à -78°C, 15h à température ambiante puis 100 mL d'eau et 50 mL d'une solution aqueuse de soude 2M sont additionnés. Après extraction par de l'acétate d'éthyle (2 x 150 mL), les phases organiques sont regroupées, séchées sur $MgSO_4$ puis concentrées sous pression réduite. L'huile obtenue est purifiée par chromatographie sur gel de silice (éluant cyclohexane/acétate d'éthyle : 4/6) pour conduire à un mélange du composé (16) et de la 3-acétylpyridine (6,7g ; 66% massique de composé (16) ; 50%) obtenu sous la forme d'une huile jaune.
RMN $^1H$ (300 MHz, $CD_3COCD_3$) : δ : 1,82 (s, 3H) ; 4,39 (s, 1H) ; 4,73-4,92 (2d ABsyst, 2H) ; 5,09 (s, 2H) ; 6,65 (m, 2H) ; 6,87 (m, 2H) ; 7,28-7,44 (m, 9H) ; 8,25 (dd, 1H) ;. 8,47 (dd, 2H) ; 8,80 (dd, 1H).

Synthèse du composé 3 : 4-(1-(pyridin-3-yl)éthyl)benzène-1,3-diol

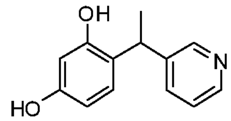

A une solution du composé (16) (4 g ; 70% massique ; 6,8 mmol, 1,0 équiv.) dans un mélange méthanol/solution aqueuse d'HCl 1M (120 mL/40 mL) est additionné du Pd/C (10%, 50% wet) (2g ; 50% massique). La suspension est agitée 4 jours sous atmosphère d'hydrogène puis filtrée sur Büchner. Le filtrat est évaporé sous pression réduite. L'huile obtenue est reprise par une solution aqueuse de soude 1M (160 mL) et lavée par de l'acétate d'éthyle (2 x 80 mL). La phase aqueuse est neutralisée par addition de $NaH_2PO_4$ solide puis filtrée sur Büchner. Le solide obtenu est lavé abondamment par de l'eau puis séché sous vide poussé pour conduire au composé 3 (0,5 g ; 35%) obtenu sous la forme d'un solide beige.
RMN $^1$H (300 MHz, DMSO) : $\delta$ : 1,48 (d, 3H) ; 4,31 (q, 1H) ; 6,18 (dd, 1H) ; 6,25 (d, 1H) ; 6,92 (d, 1H) ; 7,25 (dd, 1H) ; 7,54 (dd, 1H) ; 8,32 (ddd, 1H) ; 8,42 (d, 1H) ; 9,05 (s, 1H) ; 9,21 (s, 1H).
RMN $^{13}$C (300 MHz, DMSO) : $\delta$ : 20,47; 34,36; 102,37; 106,06; 121,87; 123,16; 127,53; 134,47; 142,27; 146,60; 148,95; 155,11; 156,50.
MS (EI): 200,1; 215,1.

Exemple 4 :

Synthèse du composé 4 : 4-(1-(thiophène-3-yl)éthyl)benzène-1,3-diol

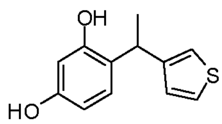

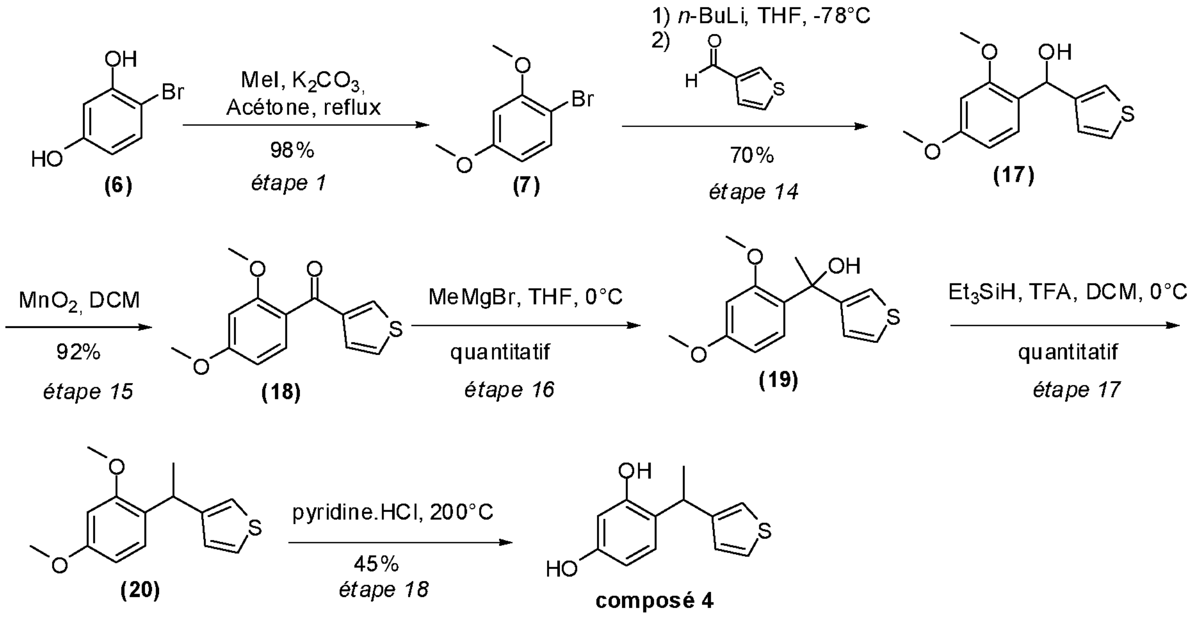


Synthèse du composé (17) : (2,4-diméthoxyphényl)(thiophène-3-yl)méthanol

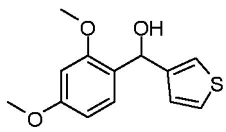

A une solution du composé (7) (3 g ; 13,8 mmol ; 1,0 équiv.) dans 90 mL de THF anhydre refroidie à -78°C est additionnée au goutte à goutte une solution de *n*-butyllithium 1,6M dans l'hexane (9,5 mL ; 15,2 mmol ; 1,1 équiv.). La solution est agitée 1h à -78°C puis le 2-formylthiophène (4,4 mL ; 49,5 mmol ; 3,6 équiv.) est additionné au goutte au goutte. La solution est agitée 3h à -78°C, 15h à température ambiante puis 150 mL d'eau sont additionnés. Après extraction par de l'acétate d'éthyle (2 x 150 mL), les phases organiques sont regroupées, séchées sur $MgSO_4$ puis concentrées sous pression réduite. L'huile obtenue est purifiée par chromatographie sur gel de silice (éluant cyclohexane/acétate d'éthyle : 10/0 à 8/2), pour conduire à un mélange du composé (17) et du thiophène-3-méthanol (2,9 g ; 83% massique de composé (17) ; 70%) obtenu sous la forme d'une huile jaune.
RMN $^1H$ (300 MHz, $CD_3COCD_3$) : $\delta$ : 3,79 (s, 3H) ; 3,83 (s, 3H) ; 4,63 (d, 1H) ; 6,12 (d, 1H) ; 6,54 (m, 2H) ; 7,01 (dd, 1H) ; 7,19 (dd, 1H) ; 7,28 (m, 1H).

<u>Synthèse du composé (18) :</u> (2,4-diméthoxyphényl)(thiophène-3-yl)méthanone

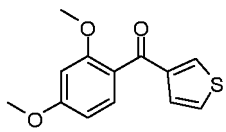

A une solution du composé (17) (2,9 g (83% massique) ; 9,6 mmol ; 1,0 équiv.) dans 100 mL de dichlorométhane est additionné du $MnO_2$ (5,0 g; 57,5 mmol; 6,0 équiv.). Le mélange réactionnel est agité 2 jours à température ambiante puis filtré sur célite. Le filtrat est concentré sous pression réduite pour conduire à un mélange du composé (17) et du composé (18). Ce mélange est remis en réaction dans les mêmes conditions pendant 2 jours. Après filtration sur célite et évaporation à sec, le résidu est purifié par chromatographie sur gel de silice (éluant cyclohexane/acétate d'éthyle : 1/0 à 9/1) pour conduire au composé (18) (2,7 g ; 92%) obtenu sous la forme d'une huile jaune.
RMN $^1H$ (300 MHz, $CD_3COCD_3$) : $\delta$ : 3,77 (s, 3H) ; 3,90 (s, 3H) ; 6,64 (dd, 1H) ; 6,68 (d, 1H) ; 7,36 (d, 1H) ; 7,46 (dd, 1H) ; 7,51 (dd, 1H) ; 7,96 (m, 1H).

<u>Synthèse du composé (19) :</u> 1-(2,4-diméthoxyphényl)-1-(thiophène-3-yl)éthanol

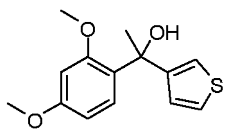

A une solution du composé (18) (2,6 g ; 10,5 mmol ; 1,0 équiv.) dans 100 mL de THF anhydre refroidie à 0°C est additionné au goutte à goutte une solution du magnésien du bromométhane 3M dans l'éther diéthylique (10,5 mL ; 31,5 mmol ; 3,0 équiv.). Le mélange réactionnel est agité 2h à 0°C puis 5 mL d'une aqueuse de soude 2M et 100 mL d'eau sont additionnés successivement. Après extraction par de l'acétate d'éthyle (2 x 100 mL), les organiques sont regroupées, séchées sur $MgSO_4$ puis concentrées sous pression réduite pour conduire au composé (18) (2,8 g ; quantitatif) obtenu sous la forme d'une huile jaune.
RMN $^1H$ (300 MHz, $CD_3COCD_3$): $\delta$ : 1,71 (s, 3H) ; 3,55 (s, 3H) ; 3,66 (s, 3H) ; 4,51 (s, 1H) ; 6,39 (m, 2H) ; 6,80 (d, 1H) ; 6,98 (dd, 1H) ; 7,12 (dd, 1H) ; 7,24 (d, 1H).

<u>Synthèse du composé (20) :</u> 3-(1-(2,4-diméthoxyphényl)éthyl)thiophène

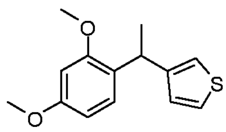

A une solution du composé (19) (2,8 g ; 10,5 mmol ; 1,0 équiv.) et de triéthylsilane (6,80 mL ; 42,0 mmol ; 4,0 équiv.) dans 60 mL de dichlorométhane anhydre refroidie à 0°C est additionné au goutte à goutte du TFA (0,85 mL; 11,5 mmol; 1,1 équiv.). Le mélange réactionnel est agité 2h à 0°C puis 30 mL d'une aqueuse saturée en $NaHCO_3$ et 100 mL d'eau sont additionnés successivement. Les phases sont séparées puis la phase aqueuse est extraite par du dichlorométhane (100 mL). Les phases organiques sont regroupées, séchées sur $MgSO_4$ puis concentrées sous pression réduite pour conduire au composé (19) (2,6 g ; quantitatif) obtenu sous la forme d'une huile jaune.
RMN $^1H$ (300 MHz, $CD_3COCD_3$) : $\delta$ : 1,39 (d, 3H) ; 3,63 (s, 3H) ; 3,69 (s, 3H) ; 4,38 (q, 1H) ; 6,31 (dd, 1H) ; 6,40 (d, 1H) ; 6,76-6,83 (m, 2H) ; 6,94 (d, 1H) ; 7,19 (dd, 1H).

<u>Synthèse du composé 4 :</u> 4-(1-(thiophène-3-yl)éthyl)benzène-1,3-diol

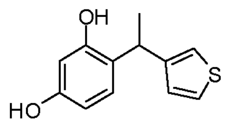

Un mélange du composé (20) (1,0 g ; 4,0 mmol ; 1,0 équiv.) et de chlorhydrate de pyridinium (30 g) est chauffé à 200°C pendant 1h30. Après refroidissement à température ambiante, de l'eau (150 mL), de l'acétate d'éthyle (75 mL) et une solution aqueuse d'HCl 2M (50 mL) sont additionnés. Les phases sont séparées puis la phase aqueuse est extraite par de l'acétate d'éthyle (2 x 100 mL). Les phases organiques sont regroupées, séchées sur $MgSO_4$ puis concentrées sous pression réduite. Le résidu est purifié par chromatographie sur gel de silice (éluant cyclohexane/acétate d'éthyle : 8/2 ; trois passages sur colonne sont nécessaires pour obtenir une pureté acceptable) pour conduire au composé 4 (0,4 g ; 45%) obtenu sous la forme d'une huile beige.
RMN $^1H$ (300 MHz, DMSO) : $\delta$ : 1,44 (d, 3H) ; 4,35 (q, 1H) ; 6,12 (dd, 1H) ; 6,27 (d, 1H) ; 6,71 (m, 1H) ; 6,89 (dd, 1H) ; 7,08 (dd, 1H) ; 7,37 (dd, 1H) ; 8,98 (s, 1H) ; 9,19 (s, 1H).
RMN $^{13}C$ (300 MHz, DMSO) : $\delta$ : 21,02; 32,11; 102,31; 106,11; 119,32; 123,00; 125,21; 127,68; 128,06; 147,81; 154,84; 156,13.
MS (EI) : 204,9; 220,0.

<u>Exemple 5 :</u>

<u>Synthèse du composé 5 : 4-(1-(thiazol-2-yl)éthyl)benzène-1,3-diol</u>

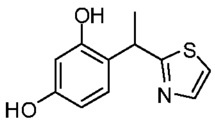

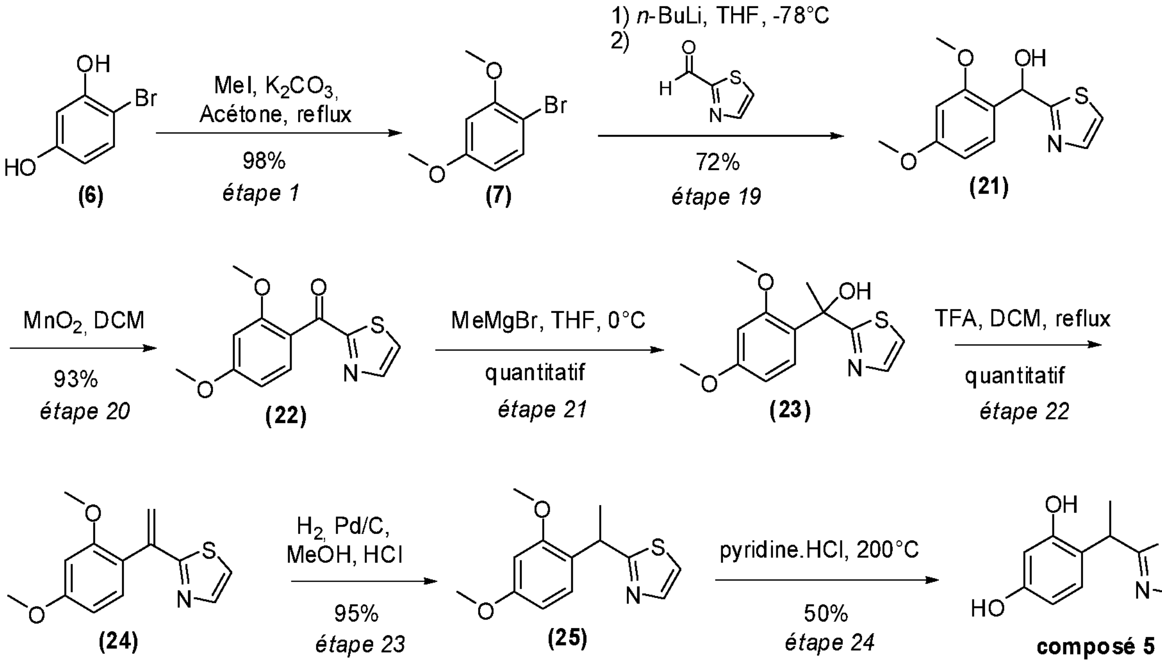

**(6)** **(7)** **(21)** **(22)** **(23)** **(24)** **(25)** **composé 5**

Synthèse du composé (21) : (2,4-diméthoxyphényl)(thiazol-2-yl)méthanol

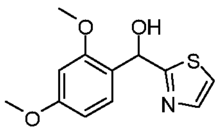

A une solution du composé (7) (3 g ; 13,8 mmol ; 1,0 équiv.) dans 90 mL de THF anhydre refroidie à -78°C est additionnée au goutte à goutte une solution de *n*-butyllithium 1,6M dans l'hexane (9,5 mL ; 15,2 mmol ; 1,1 équiv.). La solution est agitée 1h à -78°C puis le 2-formylthiophène (2,4 mL ; 37,6 mmol ; 2,0 équiv.) est additionné au goutte au goutte. La solution est agitée 3h à -78°C, 15h à température ambiante puis 150 mL d'eau sont additionnés. Après extraction par de l'acétate d'éthyle (2 x 150 mL), les phases organiques sont regroupées, séchées sur $MgSO_4$ puis concentrées sous pression réduite. L'huile obtenue est purifiée par chromatographie sur gel de silice (éluant cyclohexane/acétate d'éthyle : 7/3), pour conduire au composé (21) (2,5 g ; 72%) obtenu sous la forme d'un solide jaune.
RMN $^1H$ (300 MHz, $CD_3COCD_3$): $\delta$ : 3,80 (s, 3H) ; 3,82 (s, 3H) ; 5,25 (d, 1H) ; 6,30 (d, 1H) ; 6,52 (dd, 1H) ; 6,56 (d, 1H) ; 7,28 (d, 1H) ; 7,47 (d, 1H) ; 7,66 (d, 1H).

Synthèse du composé (22) : (2,4-diméthoxyphényl)(thiazol-2-yl)méthanone

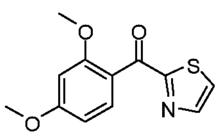

A une solution du composé (21) (2,5 g ; 9,9 mmol ; 1,0 équiv.) dans 100 mL de dichlorométhane est additionné du $MnO_2$ (5,2 g; 59,4 mmol ; 6,0 équiv.). Le mélange réactionnel est agité 20h à température ambiante puis filtré sur célite. Le filtrat est concentré sous pression réduite pour conduire au composé (22) (2,32 g ; 93%) obtenu sous la forme d'un solide jaune.
RMN $^1H$ (300 MHz, $CD_3COCD_3$): δ : 3,79 (s, 3H) ; 3,92 (s, 3H) ; 6,65 (d, 1H) ; 6,70 (d, 1H) ; 7,72 (d, 1H) ; 8,02 (m, 2H).

Synthèse du composé (23) : 1-(2,4-diméthoxyphényl)-1-(thiazol-2-yl)éthanol

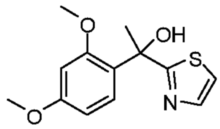

A une solution du composé (22) (2,3 g ; 9,3 mmol ; 1,0 équiv.) dans 100 mL de THF anhydre refroidie à 0°C est additionnée au goutte à goutte une solution du magnésien du bromométhane 3M dans l'éther diéthylique (9,3 mL ; 27,9 mmol ; 3,0 équiv.). Le mélange réactionnel est agité 2h à 0°C puis 5 mL d'une aqueuse de soude 2M et 100 mL d'eau sont additionnés successivement. Après extraction par de l'acétate d'éthyle (2 x 100 mL), les phases organiques sont regroupées, séchées sur $MgSO_4$ puis concentrées sous pression réduite pour conduire au composé (23) (2,42 g ; quantitatif) obtenu sous la forme d'un solide blanc.
RMN $^1H$ (300 MHz, $CD_3COCD_3$) : δ : 1,94 (s, 3H); 3,70 (s, 3H) ; 3,81 (s, 3H) ; 5,24 (s, 1H) ; 6,54 (m, 2H) ; 7,39 (d, 1H) ; 7,44 (d, 1H) ; 7,61 (d, 1H).

Synthèse du composé (24) : 2-(1-(2,4-diméthoxyphényl)vinyl)thiazole

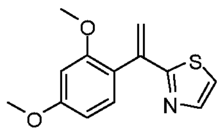

A une solution du composé (23) (2,4 g; 9,1 mmol; 1,0 équiv.) dans 70 mL de dichlorométhane anhydre est additionné au goutte à goutte du TFA (6,7 mL ; 91,0 mmol ; 10,0 équiv.). Le mélange réactionnel est agité 20h à reflux. Après refroidissement à température ambiante, 30 mL d'une aqueuse saturée en $NaHCO_3$ et 100 mL d'eau sont additionnés successivement. Les phases sont séparées puis la phase aqueuse est extraite par du dichlorométhane (100 mL). Les phases organiques sont regroupées, séchées sur $MgSO_4$ puis concentrées sous pression réduite pour conduire au composé (24) (2,5 g ; quantitatif) obtenu sous la forme d'un solide blanc.
RMN $^1H$ (300 MHz, $CD_3COCD_3$): δ : 3,69 (s, 3H) ; 3,86 (s, 3H) ; 5,32 (s, 1H) ; 6,22 (d, 1H) ; 6,58 (dd, 1H) ; 6,63 (d, 1H) ; 7,20 (d, 1H) ; 7,50 (d, 1H) ; 7,76 (d, 1H).

Synthèse du composé (25) : 2-(1-(2,4-diméthoxyphényl)éthyl)thiazole

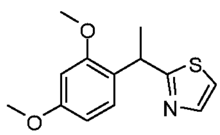

A une solution du composé (24) (2,2 g; 9,1 mmol, 1,0 équiv.) dans un mélange méthanol/ solution aqueuse d'HCl 1M (200 mL/40 mL) est additionné du Pd/C (10%, 50% wet) (1,1 g ; 50% massique). La suspension est agitée 4 jours sous atmosphère d'hydrogène puis filtrée sur Büchner. Le filtrat est évaporé sous pression réduite. L'huile obtenue est reprise par une solution aqueuse saturée en $NaHCO_3$ (100 mL) et extraite par de l'acétate d'éthyle (2 x 80 mL). Les

phases organiques sont regroupées, lavées par de la saumure (100 mL), séchées sur $MgSO_4$ puis concentrées sous pression réduite pour conduire au composé (25) (2,1 g ; 95%) obtenu sous la forme d'un solide beige.
RMN $^1H$ (300 MHz, $CD_3COCD_3$) : δ : 1,66 (d, 3H) ; 3,80 (s, 3H) ; 3,83 (s, 3H) ; 4,80 (q, 1H) ; 6,52 (dd, 1H) ; 6,57 (d, 1H) ; 7,19 (d, 1H) ; 7,37 (d, 1H) ; 7,66 (d, 1H).

Synthèse du composé 5 : 4-(1-(thiazol-2-yl)éthyl)benzène-1,3-diol

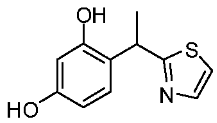

Un mélange du composé (25) (1,0 g ; 4,0 mmol ; 1,0 équiv.) et de chlorhydrate de pyridinium (30 g) est chauffé à 200°C pendant 1h30. Après refroidissement à température ambiante, de l'eau (150 mL) et de l'acétate d'éthyle sont additionnés. Les phases sont séparées puis la phase aqueuse est extraite par de l'acétate d'éthyle (2 x 100 mL). Les phases organiques sont regroupées, lavées par de la saumure (100 mL) séchées sur $MgSO_4$ puis concentrées sous pression réduite. Le résidu est purifié par chromatographie sur gel de silice (éluant cyclohexane/acétate d'éthyle : 7/3) pour conduire au composé 5 (0,45 g ; 50%) obtenu sous la forme d'un solide beige.
RMN $^1H$ (300 MHz, DMSO) : δ : 1,57 (d, 3H) ; 4,60 (q, 1H) ; 6,19 (dd, 1H) ; 6,29 (d, 1H) ; 6,90 (d, 1H) ; 7,47 (d, 1H) ; 7,65 (d, 1H) ; 9,15 (s, 1H) ; 9,41 (s, 1H).
RMN $^{13}C$ (300 MHz, DMSO) : δ : 20,47; 35,54; 102,38; 106,31; 118,91; 120,75; 128,08; 141,77; 155,22; 157,02; 176,15.
MS (EI) : 221,0.

Synthèse du composé (26) : 1-(2,4-diméthoxyphényl)-3-méthyl-1-(thiophène-2-yl)butan-1-ol

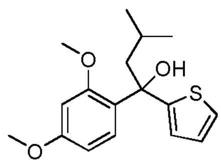

A une solution du composé (9) (2,6 g ; 10,3 mmol ; 1,0 équiv.) dans 100 mL de THF anhydre refroidie à 0°C est additionnée au goutte à goutte une solution d'isobutylmagnesium bromide 2M dans l'éther diéthylique (10,3 mL ; 30,9 mmol ; 3,0 équiv.). Le mélange réactionnel est agité 2h à 0°C puis 5 mL d'une aqueuse de soude 2M et 100 mL d'eau sont additionnés successivement. Après extraction par de l'acétate d'éthyle (2 x 100 mL), les organiques sont regroupées, séchées sur $MgSO_4$. Le résidu est purifié par chromatographie sur gel de silice pour conduire au composé (26) (avec un rendemant de 62%) obtenu sous la forme d'une huile jaune.
RMN $^1H$ (300 MHz, $CD_3COCD_3$): δ : 0,67 (d, 3H) ; 0,77 (d, 3H) ; 1,59 (m, 1H) ; 2,46 (dd, 1H) ; 3,56 (s, 3H) ; 3,65 (s, 3H) ; 4,59 (s, 1H) ; 6,39 (d, 1H) ; 6,40 (d, 1H) ; 6,68 (d, 1H) ; 6,72 (dd, 1H) ; 7,05 (dd, 1H) ; 7,37 (d, 1H).

Synthèse du composé (27) : 2-(1-(2,4-diméthoxyphényl)-3-méthylbutyl)thiophène

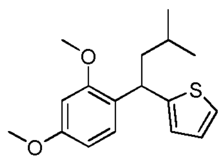

Le composé 26 (500 mg, 1,63 mmol) est solubilisé dans le Dichlorométhane (12.5ml). Le Triéthylsilane (1,04 ml, 6,52 mmol) est additionné et la solution est refroidie à - 50 °C. Du TFA (140 μL, 1,1 éq) est ajouté goutte à goutte. La solution est agitée à -50 °C pendant 2 heures est on laisse revenir à temperature ambiante. La solution est agitée

pendant la nuit. Une solution saturée de bicarbonate de sodium (30 ml) est ajoutée, suivie par de l'eau (30 ml). Les phases sont séparées et la phase aqueuse est extraite 3 fois par du dichlorométhane. Les phases organiques sont rassemblées, séchées sur $Na_2SO_4$, filtrées et concentrées. Une huile jaune est obtenue (480 mg), rendement 100%.
RMN $^1H$ (300 MHz, $CD_3COCD_3$): δ : 0,76 (d, 3H) ; 0,77 (d, 3H) ; 1,33 (m, 1H) ; 1,75 (m, 2H) ; 3,63 (s, 3H) ; 3,70 (s, 3H) ; 4,57 (t, 1H) ; 6,36 (d, 1H) ; 6,40 (d, 1H) ; 6,73 (m, 2H) ; 7,02 (d, 1H) ; 7,04 (d, 1H).

Synthèse du composé 28 : 4-(3-méthyl-1-(thiophène-2-yl)butyl)benzène-1,3-diol

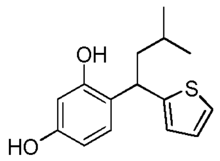

Un mélange du composé (11) (1,0 g ; 4,0 mmol ; 1,0 équiv.) et de chlorhydrate de pyridinium (30 g) est chauffé à 200°C pendant 1h30. Après refroidissement à température ambiante, de l'eau (150 mL), de l'acétate d'éthyle (75 mL) et une solution aqueuse d'HCl 2M (50 mL) sont additionnés. Les phases sont séparées puis la phase aqueuse est extraite par de l'acétate d'éthyle (2 x 100 mL). Les phases organiques sont regroupées, séchées sur $MgSO_4$ puis concentrées sous pression réduite. Le résidu est purifié par chromatographie sur gel de silice (éluant cyclohexane/acétate d'éthyle : 8/2 ; trois passages sur colonne sont nécessaires pour obtenir une pureté acceptable) pour conduire au composé 28 (avec un rendement de 20%) obtenu sous la forme d'un solide beige.
RMN $^1H$ (300 MHz, $CD_3COCD_3$): δ : 0,90 (d, 3H) ; 0,92 (d, 3H) ; 1,51 (m, 1H) ; 1,83-1,96 (m, 2H) ; 4,72 (t, 1H) ; 6,31 (d, 1H) ; 6,33 (d, 1H) ; 6,87 (m, 2H) ; 7,01 (d, 1H) ; 7,16 (dd, 1H) ; 8,05 (slarge, 1H) ; 8,30 (slarge, 1H).
RMN $^{13}C$ (75 MHz, $CD_3COCD_3$): δ :22,9 ; 23,5 ; 27,0 ; 36,9 ; 47,2 ; 103,7 ; 108,1 ; 123,8 ; 124,5 ; 127,4 ; 129,7 ; 152,1 ; 156,5 ; 157,8.
MS (ES-) : 261
Pfusion : 89,9°C

Synthèse du composé (29) : 1-(2,4-diméthoxyphényl)-1-(thiophène-2-yl)butan-1-ol

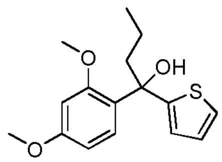

A une solution du composé (9) (2,6 g ; 10,3 mmol ; 1,0 équiv.) dans 100 mL de THF anhydre refroidie à 0°C est additionnée au goutte à goutte une solution de propylmagnesium bromide 2M dans l'éther diéthylique (10,3 mL; 30,9 mmol ; 3,0 équiv.). Le mélange réactionnel est agité 2h à 0°C puis 5 mL d'une aqueuse de soude 2M et 100 mL d'eau sont additionnés successivement. Après extraction par de l'acétate d'éthyle (2 x 100 mL), les organiques sont regroupées, séchées sur $MgSO_4$. Le résidu est purifié par chromatographie sur gel de silice pour conduire au composé (29) (avec un rendemant de 65%) obtenu sous la forme d'une huile jaune clair.
RMN $^1H$ (300 MHz, $CD_3COCD_3$): δ : 0,89 (t, 3H) ; 1,30 (m, 2H) ; 2,10 (td, 1H) ; 2,52 (td, 1H) ; 3,71 (s, 3H) ; 3,80 (s, 3H) ; 4,83 (s, 1H) ; 6,52 (d, 1H) ; 6,56 (d, 1H) ; 6,80 (d, 1H) ; 6,88 (dd, 1H) ; 7,21 (dd, 1H) ; 7,46 (d, 1H).

Synthèse du composé (30) : 2-(1-(2,4-diméthoxyphényl)butyl)thiophène

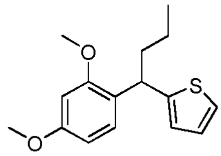

Le composé 26 (500 mg, 1,63 mmol) est solubilisé dans le Dichlorométhane (12.5ml). Le Triéthylsilane (1,04 ml, 6,52 mmol) est additionné et la solution est refroidie à - 50 °C. Du TFA (140 μL, 1,1 éq) est ajouté goutte à goutte. La solution est agitée à -50 °C pendant 2 heures est on laisse revenir à temperature ambiante. La solution est agitée pendant la nuit. Une solution saturée de bicarbonate de sodium (30 ml) est ajoutée, suivie par de l'eau (30 ml). Les phases sont séparées et la phase aqueuse est extraite 3 fois par du dichlorométhane. Les phases organiques sont rassemblées, séchées sur $Na_2SO_4$, filtrées et concentrées pour obtenir le composé 27 sous la forme d'une huile jaune.
RMN $^1H$ (300 MHz, $CD_3COCD_3$): δ : 0,79 (t, 3H) ; 1,16 (m, 2H) ; 1,91 (m, 2H) ; 3,63 (s, 3H) ; 3,68 (s, 3H) ; 4,46 (t, 1H) ; 6,34 (dd, 1H) ; 6,39 (d, 1H) ; 6,73 (m, 2H); 7,03 (m, 2H).

<u>Synthèse du composé 31 :</u> 4-(1-(thiophène-2-yl)butyl)benzène-1,3-diol

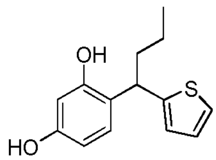

Un mélange du composé (11) (1,0 g ; 4,0 mmol ; 1,0 équiv.) et de chlorhydrate de pyridinium (30 g) est chauffé à 200°C pendant 1h30. Après refroidissement à température ambiante, de l'eau (150 mL), de l'acétate d'éthyle (75 mL) et une solution aqueuse d'HCl 2M (50 mL) sont additionnés. Les phases sont séparées puis la phase aqueuse est extraite par de l'acétate d'éthyle (2 x 100 mL). Les phases organiques sont regroupées, séchées sur $MgSO_4$ puis concentrées sous pression réduite. Le résidu est purifié par chromatographie sur gel de silice (éluant cyclohexane/acétate d'éthyle : 8/2 ; trois passages sur colonne sont nécessaires pour obtenir une pureté acceptable) pour conduire au composé 31 (avec un rendement de 18%) obtenu sous la forme d'une huile incolore.
RMN $^1H$ (300 MHz, $CD_3COCD_3$) : δ : 0,78 (t, 3H) ; 1,17 (m, 2H) ; 1,88 (m, 2H) ; 4,47 (t, 1H) ; 6,19 (dd, 1H) ; 6,26 (d, 1H); 6,74 (m, 2H); 6,86 (d, 1H); 7,03 (dd, 1H); 7,92 (slarge, 1H) ; 8,12 (slarge, 1H).
RMN$^{13}C$(75 MHz, $CD_3COCD_3$) : δ : 22,9 ;23,5 ; 26,9 ; 36,9 ; 47,2 ; 103,7 ; 108,1 ; 123 ,8 ; 124,5 ; 127,4 ; 129,7 ; 152,1 ; 156,5 ; 157,8.
MS(ES-) : [M-H]=247,3.

<u>Exemple 6:</u>

<u>Détermination de l'activité dépigmentante sur test in vitro acellulaire : test de l'inhibition de la tyrosinase)</u>

Principe :

Ce test est utilisé pour évaluer l'activité dépigmentante des molécules testées.
La tyrosinase est une enzyme limitante dans la mélanogénèse appartenant à la famille des oxydoréductases. Elle possède notamment la fonction : monophénol mono oxygénase (MPMO) et polyphénol oxydase (PPO).
La tyrosinase est synthétisée au niveau des mélanocytes. Elle est activée lors de sa migration vers les kératinocytes via les mélanosomes. Elle transforme la tyrosine en DOPA puis en dopaquinone, ce qui conduit *in fine* à une polymérisation soit une production de pigments (voir schéma ci-dessous).

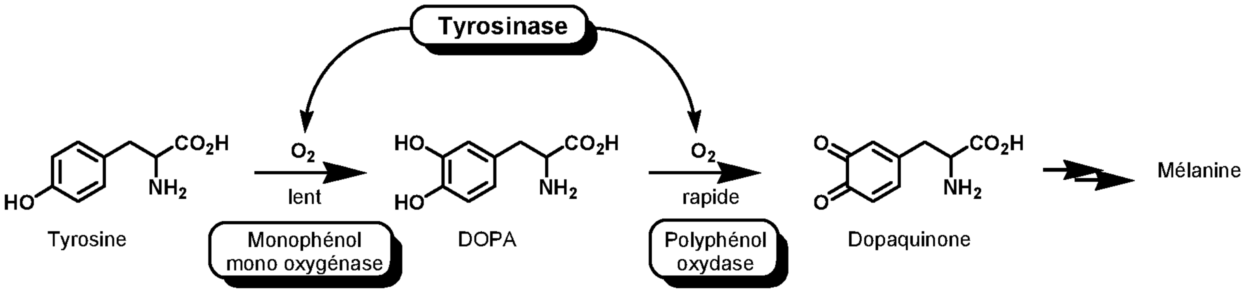


MPMO PPO

Le substrat : la L-tyrosine, est transformée par la fonction monophénol mono oxygénase en L-DOPA elle même transformée par la fonction polyphénol oxydase de la tyrosinase en dopaquinone. Cette dernière va s'auto-oxyder en dopachrome qui est mesuré en spectrophotométrie à 490 nm.

Plus précisément, c'est l'activité tyrosinase globale (MPMOg) qui est mesurée puisque c'est le dopachrome *in fine* qui est dosé. Ainsi les produits testés sur MPMOg (= mesure de l'activité sur PPO et MPMO) peuvent cumuler l'inhibition des 2 fonctions ;

- inhiber uniquement la fonction MPMO *stricto sensus* (transformation de la tyrosine en DOPA) en complément du test PPO,
- pourraient inhiber PPO uniquement

Conditions générales expérimentales :

Lecteur : Synergy HT programme : tyrosinase 280-490 cinétique : cinétique sur 45 min, lecture à t = 10minutes, Essais en 96 puits transparents, Tampon Phosphate - pH 6.8, Enzyme : mushroom tyrosinase - Sigma = T-3824Substrat : L-Tyrosine - Sigma = T-3754, Témoin positif : Acide kojique (AK) - fluka = 60890 (inhibiteur de référence)

Molécules de référence pour le test :

Acide kojique : 9μM< IC 50 <20μM (PPO), 3μM< IC 50 <7μM (MPMO)
Vitamine C : 20μM< IC 50 <40μM (PPO)
Glutathion réduit : 55% inhibition à 25μM (PPO), IC 50 = 1-2 μM (MPMO)
Hydroquinone : IC 50 = 3-4 μM (MPMO)
Arbutine : 57% inhibition à 88μM (MPMO)

Ces molécules exogènes sont connues pour réguler négativement la mélanogenèse. L'hydroquinone inhibe la synthèse de mélanine en se présentant comme substrat de la tyrosinase afin de détourner son activité. L'arbutine contenant de l'hydroquinone agit de la même manière. L'acide kojique diminue l'activité de la tyrosinase en inhibant l'hyperpigmentation induite par les UV. La vitamine C inhiberait la tyrosinase mais se comporterait également comme un réducteur puissant en empêchant la coloration de la mélanine par oxydation. La vitamine A diminue l'expression de la tyrosinase.

<u>Résultats :</u>

| Exemples | Structure | Résultats d'activités dépigmentantes Tyrosinase (IC50 en μM) |
|---|---|---|
| Composé 1 | 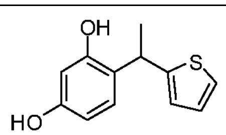 | 0,09 |

(suite)

| Exemples | Structure | Résultats d'activités dépigmentantes Tyrosinase (IC50 en $\mu$M) |
|---|---|---|
| Composé 2 | 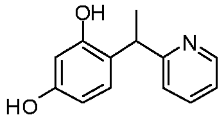 | 0,4 |
| Composé 3 | 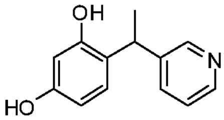 | 0,2 |
| Composé 4 | 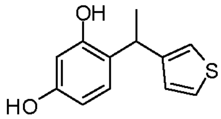 | 0,08 |
| Composé 5 | 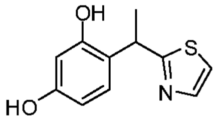 | 1,5 |
| Acide Kojique | 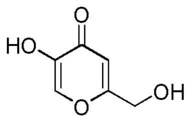 | 15 |
| 4-butyl-résorcinol | 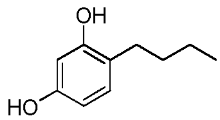 | 1 |
| Arbutine | 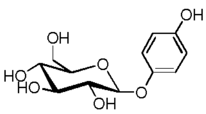 | 90 |
| Dérivé Résorcinol Unigen | 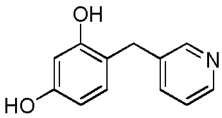 | 2 |
| Dérivé Résorcinol Unigen | 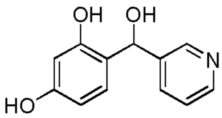 | 4,9 |
| 4-(1-phenyléthyl)benzène-1,3-diol | 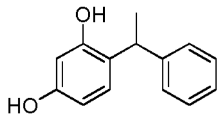 | 0,1 |

Les tests comparatifs menés sur les composés de l'art antérieur Unigen (WO2010/011630) ont permis de démontrer la supériorité d'activité des composés selon l'invention. Plus particulièrement, nous pouvons noter que le composé 3 selon l'invention, le plus proche chimiquement, a une IC50 significativement plus faible (0,2 $\mu$M vs 2 et 4,9 $\mu$M).

Par rapport au composé 4-(1-phényléthyl)benzène-1,3-diol de l'art antérieur même si les structures des composés selon l'invention peuvent sembler assez proches (voir le composé 3), le fait d'avoir une unité pyridine à la place d'un phényle permet de pouvoir solubiliser l'actif en solution aqueuse acide, alors que le composé 4-(1-phényléthyl)benzène-1,3-diol) lui est insoluble en phase aqueuse.

Nous avons ici une aide potentielle non négligeable pour la mise en formule de l'actif selon l'invention sous

forme sel ; ou lors de la purification de l'actif lui-même comme utilisé lors de sa synthèse (voir exemple 3 : synthèse du composé 3).

Exemple 7 :

Test du dosage de la mélanine dans des cellules B16-F10 :

Principe :

Il s'agit d'un test de mesure de la synthèse de mélanine par dosage colorimétrique sur une lignée cellulaire de mélanomes murins: la lignée B16-F10. Ce test permet d'évaluer le pouvoir dépigmentant de principes actifs comme l'acide kojique ou l'arbutine.

Les cellules B16-F10 sont ensemencées dans des plaques 96 puits dans du milieu DMEM supplémenté de SVF (sérum de veau foetal), et incubées 24 heures à 37°C, 5%$CO_2$. Les cellules sont ensuite stimulées à l'$\alpha$-MSH 0.1$\mu$M (pour stimuler la synthèse de mélanine, la stimulation observée est d'environ 150%) et traitées 72 heures avec les actifs à tester. Chaque concentration d'actif est testée au moins en triplicata. La mélanine totale suivie de la mélanine intracellulaire dissoute dans du tampon de lyse sont alors dosées par lecture d'absorbance à 405 nm. Les protéines totales sont dosées dans le lysat et les résultats sont exprimés en mg mélanine/mg protéines. Le pourcentage d'activité est calculé comme suit :

$$\% \text{ d'activité} = \frac{\text{Moyenne normalisée du témoin} - \text{Moyenne normalisée du traité}}{\text{Moyenne normalisée du témoin}} \times 100$$

Une valeur négative indique une inhibition, alors qu'une valeur positive indique une induction de la synthèse de mélanine.

Conditions générales expérimentales :

Matériel :

- Incubateur cellulaire à CO2 (Heraeus), Etuve, Centrifugeuse (Heraeus), Hotte à flux d'air laminaire, Plaques 96 puits à fond plat transparent - Falcon, Cônes stériles - Treff Lab, Polylabo,Mithras LB940 (Berthold Technologies)- 154/MIPA/003

Matériel biologique :

- Lignée cellulaire B16-F10 entre P10 et P20 (mélanocytes murins) (ATCC, CRL-6475)

Réactifs :

DMEM sans rouge de phénol (GIBCOBRL, 31053-028), Glutamax-I Supplément à 200mM (GIBCOBRL, 35050-038), D-PBS (GIBCOBRL, 14190-094), Sérum de veau foetal (Invitrogen, 10270-098), Trypsine-EDTA (GIBCOBRL, 25300-054), NaOH (Sigma, S8045-500G), DMSO (Sigma, 471267-1L), Nle, Phe - Melanocyte Stimulating Hormon (Sigma, M-8764), Mélanine (Sigma, M-0418), BCA-COPPER (SIGMA, B9643 et C2284), BSA (SIGMA, P0914)

Résultats :

| Exemple | Structure | Dosage Mélanine dans des souches B16F10 (%) |
|---|---|---|
| Composé 1 | 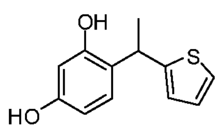 | - 50% à 20 $\mu$M |

(suite)

| Exemple | Structure | Dosage Mélanine dans des souches B16F10 (%) |
|---|---|---|
| Composé 2 | 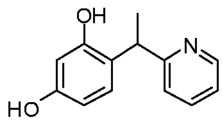 | - 26% à 20 μM |
| Composé 3 | 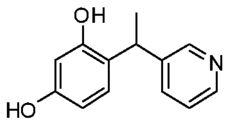 | - 52% à 20 μM |
| Composé 4 | 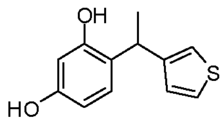 | - 52% à 20 μM |
| Composé 5 | 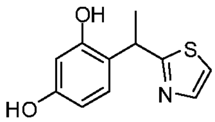 | - 33% à 20 μM |
| Composé 28 | 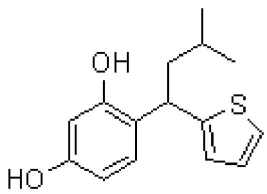 | -52 % à 10 μM |
| Composé 31 | 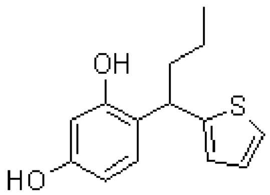 | -50 % à 10 μM |
| Acide Kojique | 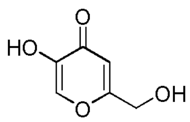 | IC50 = 2400 μM |
| Arbutine | 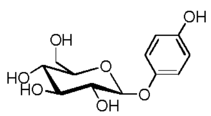 | IC50 = 158 μM |

Il semble important de préciser que des valeurs négatives de pourcentage indiquent une inhibition de la synthèse de mélanine, et qu'en revanche, une valeur positive indique une induction de cette dernière.

Les composés1 à 5, 28 et 31 se révèlent donc être de forts inhibiteurs de la synthèse de mélanine dans des souches définies de type B 16F 10.

<u>Exemple 8 :</u>

Test pour l'étude de la capacité antioxydante par chemiluminiscence (Photochem analytic Jena) Principe :

Ce test est utilisé pour déterminer la capacité antioxydante des molécules. C'est une méthode qui génère des radicaux libres par un signal photochimique. L'intensité de l'oxydation est 1000 fois supérieure à celles obtenues en conditions normales.

La détection se fait par chemiluminescence. Elle permet l'évaluation de molécules ou extraits anti-oxydants hydrosolubles et liposolubles.

Les résultats sont exprimés respectivement en quantité équivalente de vitamine C ou de Trolox (acide 6-Hydroxy-2,5,7,8-tétraméthylchroman-2-carboxylique). La sensibilité est de l'ordre de la nanomole.

L'activité anti-oxydante étudiée dans ce test représente la capacité à piéger spécifiquement les anions superoxydes par chemiluminescence.

Les résultats quantifiés sont exprimés en équivalent Trolox (standard) soit en « µg de produit pour 1µg de Trolox ». Cela signifie qu'il faut une quantité x d'échantillon pour obtenir une activité équivalente à l'activité détectée pour 1µg de standard. C'est un pouvoir anti-oxydant relatif à une référence, cela nous affranchit de la concentration testée.

- Génération de radicaux libres oxygénée :

Le radical superoxide : $O_2^{°-}$ est généré par une réaction photochimique :

$$L + hv\ (UV) + O_2 \rightarrow L^*\ O_2 \rightarrow L^{°+} + O_2^{°-}$$

L*: luminol à l'état excité
*L°+ : radical luminol*

- Détection du signal :

Une partie des anions superoxydes est quenchée par les anti-oxydants. Les radicaux libres restants sont quantifiés par chemiluminescence.

$$L^{°+} + O_2^{°-} \rightarrow N2 + AP^{*2-} \rightarrow AP^{2-} + hv\ (luminescence)$$

$AP^{*2-}$ : *aminophtalate à l'état excité*

| Nom | Conditions | Photosensibilisant | Antioxydant |
|---|---|---|---|
| Blanc | 100% $O_2^{°-}$ générés | + | - |
| Standards | Gamme standard: De 1 à 3 nmol | + | Vitamine C Ou trolox |
| Essai | +/- $O_2^{°-}$ générés | + | Molécule x à tester |

L'échelle pour interpréter les résultats est la suivante :

| *Produits* | µg éch pour 1µg de Trolox | Activité |
|---|---|---|
| Vitamine C | 0.1 à 3.0 | Très bonne |
| BHT | 3.01 à 50 | Bonne |
| Cystéine | 50.1 à 1000 | Moyenne |
| Albumine | > 1000 | Faible |
| Acide Lipoïque | NEGATIF | Nulle |

La plupart des composés présentent des résultats comparables à la vitamine C. Tous les composés montrent des résultats inférieurs à 1000µg de trolox (301µg étant le plus faible résultat obtenu avec le résorcinol sulfone) ; ils ont donc tous une activité anti-oxydante intéressante.

Résultats :

| *Exemple* | *Structure* | µg d'échantillon pour 1 µg de trolox |
|---|---|---|
| Composé 1 | 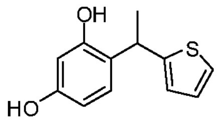 | 13,7 |
| Composé 2 | 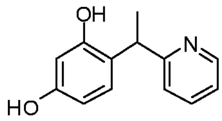 | 10,4 |
| Composé 3 | 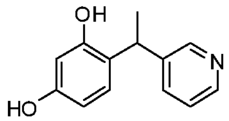 | 8,7 |
| Composé 4 | 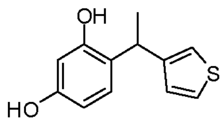 | 15,4 |
| Composé 5 | 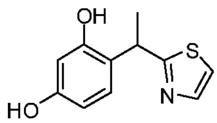 | 11,7 |

<u>Exemple 9 : Composition dépigmentante :</u>

| Ingrédients (noms commerciaux) | INCI désignation | Pourcentage en poids | Fonction |
|---|---|---|---|
| I. Eau purifiée | eau | QSP 100% | |
| Hydrolite 5 | Pentylène Glycol | 3 | Humectant, Conservateur |
| EDTA, 2Na | Disodium EDTA | 0.1 | Agent complexant |
| Microcare PM4 | Phenoxyethanol-Parabène | 0.8 | Conservateurs |
| PCL Hydrosoluble | Trideceth-9 & PEG-5 Éthylhexanoate | 1.5 | Emollient aqueux |
| II. Pemulen TR-1 | Acrylates/C 10-30 AlkylCrosspolymer | 0.5 | Gélifiant, agent stabilisant |
| III. Stearine TP | acide stéarique | 2 | Emulsionnant, facteur de consistance |
| PCL Liquide | Cetearyléthylhexanoate & Isopropylmyristate | 3 | Emollient |
| DC200 | Diméthicone | 0.3 | Emollient |
| Myritol 318 | Triglycérides caprique ou caprylique | 3 | Emollient |
| Primol 352 | paraffine liquide | 2 | Emollient |
| IV. Actif Dépigmentant | Composés 1 à 5 au choix | 0,5 | Actif |
| V. Soude | NaOH | 0,08 | Ajusteur de pH |

Dans une telle composition, le pourcentage du principe actif peut varier entre 0,01% et 10% en poids et de préférence de 0,1% à 5% en poids par rapport au poids total de la composition.

L'invention vise également les compositions pharmaceutiques ou cosmétiques comprenant au moins un des composés de formule (I) en association avec au moins un excipient pharmaceutiquement ou cosmétiquement acceptable

Selon un mode de réalisation particulier de l'invention, les compositions pharmaceutiques ou cosmétiques comprennent au moins un des composés de formule (I) en association avec au moins un excipient pharmaceutiquement ou cosmétiquement acceptable et une phase grasse.

Selon un mode de réalisation particulier de l'invention, les compositions pharmaceutiques ou cosmétiques comprennent au moins un des composés de formule (I) en association avec au moins un excipient pharmaceutiquement ou cosmétiquement acceptable et un émollient.

La présente invention concerne une composition cosmétique dépigmentante de la peau et/ou des cheveux et/ou des poils caractérisée en ce qu'elle comprend au moins un composé de formule (I).

L'invention vise également une composition cosmétique anti-vieillissement de la peau, caractérisée en ce qu'elle comprend au moins un composé de formule (I).

La composition selon l'invention peut comprendre en outre des adjuvants cosmétiques classiques notamment choisis parmi les phases grasses, les solvants organiques, les épaississants, les adoucissants, les opacifiants, les stabilisants, les émollients, les agents anti-mousse, les agents hydratants, les parfums, les conservateurs comme les parabens, les polymères, les charges, les séquestrants, les bactéricides, les absorbeurs d'odeur, les agents alcalinisants ou acidifiants, les tensio-actifs, les anti-radicaux libres, les antioxydants, les vitamines E et C, les $\alpha$-hydroxyacides, ou des eaux thermales ou tout autre ingrédient habituellement utilisé en cosmétique, en particulier pour la fabrication de compositions de ce type.

La composition selon l'invention peut en outre comprendre une phase grasse. La phase grasse peut être constituée par une huile ou une cire ou leurs mélanges, et comprendre également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non comme la dimethicone ; les isoparaffines, les polyoléfines, les huiles fluorées et perfluorées. De même, les cires peuvent être choisies parmi les cires animales, fossiles, végétales ou de synthèse telles que les cires d'abeille, les cires de candelila, les cires de canauba, le beurre de karité, de la cire de pétrole (ou cire microcristalline), la paraffine, et leurs mélanges.

La composition selon l'invention peut comprendre en outre un polyol miscible à l'eau à la température ambiante (environ 25°C), notamment choisi parmi les polyols ayant notamment de 2 à 20 atomes de carbones, de préférence ayant de 2 à 10 atomes de carbone, et préférentiellement ayant de 2 à 6 atomes de carbones, tels que la glycérine ; les dérivés de glycol tel que le propylène glycol, le butylène glycol, le pentylène glycol, l'hexylène glycol, le dipropylène glycol, le diéthylène glycol ; les éthers de glycol tels que les éthers d'alkyle en $C_1$-$C_4$ de mono-, di- ou tri-propylène glycol, les éthers d'alkyle en $C_1$-$C_4$ de mono-, di- ou tri-éthylène glycol ; et leurs mélanges.

La composition selon l'invention peut également comprendre des agents épaississant ou des agents de modification de la rhéologie, tels que par exemple des uréthanes non ioniques éthoxylés hydrophobiquement modifiés, des acides polycarboxyliques épaississants tels que des copolymères d'acrylates/stéareth-20 méthacrylate, des carbomères, des copolymères d'acrylates réticulés et leurs mélanges.

La composition selon l'invention peut également comprendre des acides et des bases permettant d'ajuster la zone de pH de ladite composition. Les bases peuvent être minérales (soude, potasse, ammoniaque, etc.) ou organiques telles que la mono-, di- ou tri-éthanolamine, un aminométhylpropanediol, la N-méthyl-glucamine, les acides aminés basiques comme l'arginine et la lysine, et leurs mélanges.

La composition selon l'invention peut également comprendre des agents de conditionnement de la peau. Des exemples d'agents de conditionnement de la peau comprennent, sans y être limité, des émulsifiants anioniques, cationiques et non ioniques tels que le sulfate de lauryle de sodium, le sulfosuccinate de dioctyle de sodium, le stéarate de sodium, l'ester de sorbitane ; des acides gras éthoxylés ; des alcools gras éthoxylés tels que le trideceth-9 et le PEG-5 éthylhexanoate ; l'acide stéarique ; tout autre émulsifiant et agent de conditionnement connu de l'homme du métier ; et leurs mélanges.

La composition selon l'invention peut en outre contenir d'autres principes actifs conduisant à un effet complémentaire.

La composition selon l'invention peut être présentée sous n'importe quelle forme appropriée pour une application topique, notamment sur la peau et/ ou les cheveux. En particulier, elles peuvent se présenter sous la forme d'émulsions obtenues par la dispersion d'une phase grasse dans une phase aqueuse, par exemple une émulsion huile-dans-eau ou eau-dans-huile ou multiple, ou sous la forme d'un gel ou d'un produit anhydre liquide, pâteux ou solide, ou sous la forme d'une dispersion en présence de sphérules. La composition selon l'invention peut également être moins fluide et se présenter sous la forme d'une crème blanche ou colorée, d'une pommade, d'un lait, d'une lotion, d'un sérum, d'une pâte, d'un masque, d'une poudre, d'un stick solide, ou éventuellement d'un aérosol, d'une mousse ou d'un spray.

## Revendications

**1.** Composé de formule générale (I)

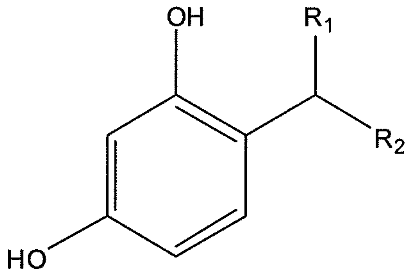

(I)

**caractérisé en ce que** :

- R1 est un groupement méthyle,
- et R2 représente un monocycle hétéro aromatique, azoté, soufré ou oxygéné, de préférence à 5 ou 6 chaînons

**2.** Composé selon la revendication 1, **caractérisé en ce que** R2 est choisi parmi le groupe constitué d'un hétérocycle pyridine, thiophène et thiazole.

**3.** Composé selon l'une des revendications let 2, **caractérisé en ce que** R1 est un groupement méthyle et R2 est choisi dans le groupe constitué du 3-pyridyl, 4-pyridyl, 2-furyl, 3-furyl, 2-thiophényl, 3-thiophényl, et 2-thiazolyl.

**4.** Composé selon la revendication 1, **caractérisé en ce qu'**il est choisi parmi l'un des composés suivants :

- 4-(1-(pyridin-2-yl)éthyl)benzène-1,3-diol,
- 4-(1-(pyridin-3-yl)éthyl)benzène-1,3-diol,
- 4-(1-(pyridin-4-yl)éthyl)benzène-1,3-diol,
- 4-(1-(thiophène-2-yl)éthyl)benzène-1,3-diol,
- 4-(1-(thiophène-3-yl)éthyl)benzène-1,3-diol,
- 4-(1-(thiazol-2-yl)éthyl)benzène-1,3-diol,
- 4-(1-(1-méthyl-1H-benzo[d]imidazol-2-yl)éthyl)benzène-1,3-diol,
- 4-(1-(1-méthyl-1H-indol-2-yl)éthyl)benzène-1,3-diol,
- 4-(1-(benzo[b]thiophène-2-yl)éthyl)benzène-1,3-diol,
- 4-(1-(thiophène-2-yl)butyl)benzène-1,3-diol,
- 4-(3-méthyl-1-(thiophène-2-yl)butyl)benzène-1,3-diol,.

**5.** Composé tel que défini selon l'une quelconque des revendications 1 à 4, pour son utilisation en tant que principe actif cosmétique.

**6.** Composé tel que défini selon l'une quelconque des revendications 1 à 4 pour son utilisation, en tant que médicament.

**7.** Composé tel que défini selon l'une quelconque des revendications 1 à 4 pour son utilisation, en tant que principe actif dépigmentant.

**8.** Composé tel que défini selon l'une quelconque des revendications 1 à 4, pour son utilisation en tant que principe actif antioxydant.

**9.** Composé tel que défini selon l'une quelconque des revendications 1 à 4, pour son utilisation en tant qu'agent antivieillissement de la peau.

**10.** Composé tel que défini selon l'une quelconque des revendications 1 à4, pour son utilisation en tant qu'agent désinfectant de la peau.

**11.** Composition pharmaceutique ou cosmétique, **caractérisée en ce qu'**elle comprend à titre de principe actif au moins un composé de formule (I) tel que défini selon l'une quelconque des revendications 1 à 4 en association avec un excipient pharmaceutiquement ou cosmétiquement acceptable.

**12.** Composition pharmaceutique ou cosmétique selon la revendication 11, **caractérisée en ce que** la quantité de composé de formule (I) varie entre 0,01% et 10% en poids par rapport au poids total de la composition.

## Patentansprüche

**1.** Verbindung der allgemeinen Formel (I):

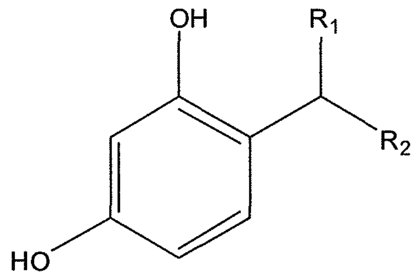

(I)

**dadurch gekennzeichnet, dass**:

- R1 eine Methylgruppe ist,
- und R2 einen Stickstoff, Schwefel oder Sauerstoff enthaltenden heteroaromatischen Monocyclus, vorzugsweise mit 5 oder 6 Ringen, darstellt.

**2.** Verbindung nach Anspruch 1, **dadurch gekennzeichnet, dass** R2 aus der Gruppe ausgewählt ist, die von einem Pyridin-, Thiophen- und Thiazol-Heterocyclus gebildet ist.

**3.** Verbindung nach einem der Ansprüche 1 und 2, **dadurch gekennzeichnet, dass** R1 eine Methylgruppe ist und R2 aus der Gruppe ausgewählt ist, die von 3-Pyridyl, 4-Pyridyl, 2-Furyl, 3-Furyl, 2-Thiophenyl, 3-Thiophenyl und 2-Thiazolyl gebildet ist.

**4.** Verbidung nach Anspruch 1, **dadurch gekennzeichnet, dass** sie aus einer der folgenden Verbindungen ausgewählt ist:

- 4-(1-(Pyridin-2-yl)ethyl)benzen-1,3-diol,
- 4-(1-(Pyridin-3-yl)ethyl)benzen-1,3-diol,
- 4-(1-(Pyridin-4-yl)ethyl)benzen-1,3-diol,
- 4-(1-(Thiophen-2-yl)ethyl)benzen-1,3-diol,
- 4-(1-(Thiophen-3-yl)ethyl)benzen-1,3-diol,
- 4-(1-(Thiazol-2-yl)ethyl)benzen-1,3-diol,
- 4-(1-(1-Methyl-1H-benzo[d]imidazol-2-yl)ethyl)benzen-1,3-diol,
- 4-(1-(1-Methyl-1H-indol-2-yl)ethyl)benzen-1,3-diol,
- 4-(1-(Benzo[b]thiophen-2-yl)ethyl)benzen-1,3-diol,
- 4-(1-(Thiophen-2-yl)butyl)benzen-1,3-diol,
- 4-(3-Methyl-1-(thiophen-2-yl)butyl)benzen-1,3-diol.

**5.** Verbindung nach einem der Ansprüche 1 bis 4 für ihre Verwendung als kosmetischer Wirkstoff.

**6.** Verbindung nach einem der Ansprüche 1 bis 4 für ihre Verwendung als Arzneimittel.

**7.** Verbindung nach einem der Ansprüche 1 bis 4 für ihre Verwendung als depigmentierender Wirkstoff.

**8.** Verbindung nach einem der Ansprüche 1 bis 4 für ihre Verwendung als antioxidativer Wirkstoff.

**9.** Verbindung nach einem der Ansprüche 1 bis 4 für ihre Verwendung als Wirkstoff gegen Hautalterung.

**10.** Verbindung nach einem der Ansprüche 1 bis 4 für ihre Verwendung als Wirkstoff zum Desinfizieren der Haut.

**11.** Pharmazeutische oder kosmetische Zusammensetzung, **dadurch gekennzeichnet, dass** sie als Wirkstoff mindestens eine Verbindung der Formel (I) nach einem der Ansprüche 1 bis 4 in Kombination mit einem pharmazeutisch oder kosmetisch akzeptablen Hilfsstoff umfasst.

**12.** Pharmazeutische oder kosmetische Zusammensetzung nach Anspruch 11, **dadurch gekennzeichnet, dass** die Menge der Verbindung der Formel (I) zwischen 0,01 und 10 Gew.-% im Verhältnis zum Gesamtgewicht der Zusammensetzung schwankt.

## Claims

**1.** A compound of general formula (I)

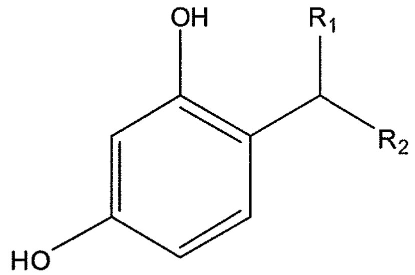

(I)

**characterized in that**:

- $R_1$ is a methyl group,
- $R_2$ is a nitrogen-, sulfur- or oxygen-containing heteroaromatic single-ring, preferably with 5 or 6 members.

**2.** The compound according to claims 1, **characterized in that** $R_2$ is selected from the group composed of a pyridine, thiophene and thiazole heterocycle.

**3.** The compound according to one of claims 1 and 2, **characterized in that** $R_1$ is a methyl group and $R_2$ is selected from the group composed of 3-pyridyl, 4-pyridyl, 2-furyl, 3-furyl, 2-thiophenyl, 3-thiophenyl, and 2-thiazolyl.

**4.** The compound according to claim 1, **characterized in that** it is selected from one of the following compounds:

- 4-(1-(pyridin-2-yl)ethyl)benzene-1,3-diol;
- 4-(1-(pyridin-3-yl)ethyl)benzene-1,3-diol;
- 4-(1-(pyridin-4-yl)ethyl)benzene-1,3-diol;
- 4-(1-(thiophene-2-yl)ethyl)benzene-1,3-diol;
- 4-(1-(thiophene-3-yl)ethyl)benzene-1,3-diol;
- 4-(1-(thiazol-2-yl)ethyl)benzene-1,3-diol;
- 4-(1-(1-methyl-1H-benzo[d]imidazol-2-yl)ethyl)benzene-1,3-diol;
- 4-(1-(1-methyl-1H-indol-2-yl)ethyl)benzene-1,3-diol;
- 4-(1-(benzo[b]thiophene-2-yl)ethyl)benzene-1,3-diol;
- 4-(1-(thiophene-2-yl)butyl)benzene-1,3-diol;
- 4-(3-methyl-1-(thiophene-2-yl)butyl)benzene-1,3-diol.

**5.** The compound as defined according to any one of claims 1 to 4, for use as a cosmetic active ingredient.

**6.** The compound as defined according to any one of claims 1 to 4, for use as a drug.

**7.** The compound as defined according to any one of claims 1 to 4, for use as a depigmenting active ingredient.

**8.** The compound as defined according to any one of claims 1 to 4, for use as an antioxidant active ingredient.

**9.** The compound as defined according to any one of claims 1 to 4, for use as an anti-aging agent of the skin.

**10.** The compound as defined according to any one of claims 1 to 4, for use as a disinfectant agent of the skin.

**11.** A pharmaceutical or cosmetic composition, **characterized in that** it includes as active ingredient at least one of the compounds of formula (I) such as defined according to any one of claims 1 to 4 in combination with a pharmaceutically or cosmetically acceptable excipient.

**12.** A pharmaceutical or cosmetic composition according to claim 11, **characterized in that** the quantity of the compound of formula (I) varies between 0.01% and 10% by weight in relation to the total weight of the composition.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2010011630 A
- US 2010016347 A
- US 2007098655 A